**Europäisches Patentamt**

**European Patent Office**

**Office européen des brevets**

(19)

(11) **EP 0 121 794 B2**

(12) # NEW EUROPEAN PATENT SPECIFICATION

(45) Date of publication and mention
of the opposition decision:
**27.03.1996 Bulletin 1996/13**

(45) Mention of the grant of the patent:
**06.12.1989 Bulletin 1989/49**

(21) Application number: **84102590.1**

(22) Date of filing: **09.03.1984**

(51) Int. Cl.$^6$: **A61N 1/36**

(54) **Pacemaker with switchable circuits and method of operation of same**

Schrittmacher mit schaltbaren Schalteinheiten und Verfahren zu seinem Gebrauch

Stimulateur cardiaque avec des circuits commutables et procédé pour son opération

(84) Designated Contracting States:
**CH DE FR GB IT LI NL**

(30) Priority: **14.03.1983 US 475024**

(43) Date of publication of application:
**17.10.1984 Bulletin 1984/42**

(73) Proprietor: **VITATRON MEDICAL B.V.
NL-6950 AB Dieren (NL)**

(72) Inventors:
• **Mensink, Kornelis A.
Brummen (NL)**
• **Brouwer, Hendrik L.
Dieren (NL)**

(74) Representative: **Ebbinghaus, Dieter, Dipl.-Ing. et al
Patentanwälte v. Füner, Ebbinghaus, Finck
Postfach 95 01 60
D-81517 München (DE)**

(56) References cited:
EP-A- 0 006 207          EP-A- 0 007 189
EP-A- 0 017 848          EP-A- 0 057 944
DE-A- 2 731 883          US-A- 4 381 786

• J.MARKUS: "Sourcebook of Electronic Circuits",
McGraw-Hill Book Company, New York 1968,
page 507

EP 0 121 794 B2

**Description**

This invention lies in the area of cardiac pacers and method of operation of same, with means for varying operating characteristics of one or more circuits and, in particular, pacers and other implantable devices with microprocessor control for switching the characteristics of one or more circuits on a predetermined basis.

It is known that pacemakers, and other implanted devices generally, have a need to respond to different types of sensed signal information. Thus, for example, in the pacemaker embodiment, where the implanted device operates on a cyclical basis, it may be desirable to change the operating characteristics of the circuit or circuits which sense the patient-generated signals, either within each cycle of operation, or over a period of time. In the prior art it is well known that parameters can be programmed externally, but such programming does not meet the objective of this invention for changing one or more circuit characteristics during each cycle, nor does programming provide for automatic response to sensed patient conditions.

The construction of a demand pacemaker is well known in the art. See, for example, U.S. Patent No. 4,228,803. Such a pacemaker incorporates an input amplifier for sensing the natural heart signals. It has been conventional in the art to blank such an input amplifier during the generation of a stimulus pulse. See also J. Markus, Source Book of Electronic Circuits, McGraw Hill Book Company, New York, 1968, p. 507, "Pulsed-Transducer Meter Protection," describing an amplifier associated with a filter which can be blanked by an adequate control signal. Furthermore, the pacemaker has utilized micro-processor-based circuitry, which can be used for controlling of delivery of required blanking pulses. See, for example, EP-A 0 017 848. However, the prior art does not show changing the frequency response of an input amplifier in an implantable device such as a pacemaker, during the measurement on a cyclical basis.

While it is known that programmable circuits are available in other art areas, there has been no use or incorporation of controllable or switchable circuits into implantable devices for more efficient use thereof. For devices such as cardiac pacemakers, muscle stimulators, automatic drug dispensers, etc., there is a substantial need for the advantages to be gained by proper use of automatic switching, or by controlling the characteristics of circuits, e.g. changing filter characteristics. By use of a microprocessor incorporated with the implanted device, it is possible to optimize circuit operation in a manner which has heretofore been unavailable to the art.

Summary of the invention

It is an object to provide a cardiac pacemaker having a controllable input amplifier circuit which can be switched on a periodic basis to provide different response characteristics during different portions of each cycle. In particular, it is an object to provide a cardiac pacemaker which is controlled to change the frequency response of its input amplifier on a cyclical basis, so as to provide a different response for detection of both QRS complexes and the T wave portion of the sensed heartbeat signal.

It is another object of this invention to provide microprocessor control for changing the circuit characteristics of one or more circuits within a cycle of a device such as a cardiac pacemaker which operates on a cyclic basis.

An advantage of this invention is that it provides microprocessor control of a sense amplifier used for detection of evoked responses and T waves in a cardiac pacer, as part of a pacer system for optimizing pacer rate control.

It is another object of this invention to provide microprocessor control of circuit characteristics, such as the frequency setting of an amplifier filter for amplifying sensed cardiac signals, or for controlling sensitivity of an input amplifier, over a plurality of cardiac cycles.

In accordance with the present invention the above objects are solved by a cardiac pacemaker as described in claim 1.

A preferred further development of the cardiac-pacemaker of the present invention is described in claim 2.

Brief description of the drawings

Fig. 1 is a circuit diagram of a programmable filter and sense amplifier for use in a cardiac pacemaker.

Fig. 2A is a portion of a flow diagram showing microprocessor control of a pacer system which incorporates a change of one or more circuits during each cycle of operation.

Fig. 2B is a portion of a flow diagram showing microprocessor control of a cardiac pacer which adjusts the pacing rate as a function of sensed stimulus-T wave times, incorporating control of one or more circuit characteristics.

Fig. 2C is a flow diagram of microprocessor control of an implanted device, such as a cardiac pacemaker, which operates cyclically, incorporating control of circuit characteristics effected over a time period of more than one cycle.

Description of the preferred embodiments

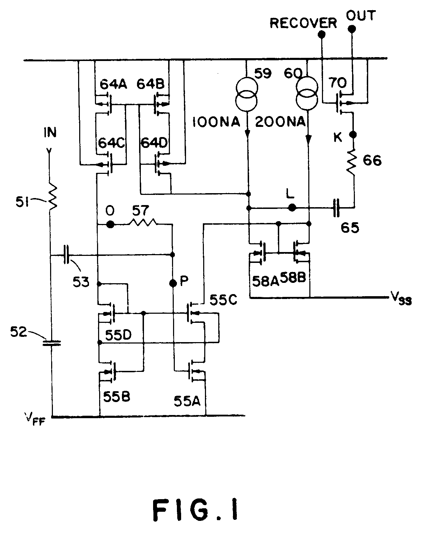

Referring now to Fig. 1, there is shown a circuit diagram of a QRS detection amplifier adapted for use in a cardiac pacemaker, having a programmable filter. In general, such a programmable filter may use one or more time constant-determining networks, e.g. resistor and capacitor, transconductance and capacitor, frequency-controlled charge switch and capacitor, etc. A switching means, preferably controlled by a logic sequencer such as provided by a microprocessor, turns on and off one or more of the time constant networks, or changes the switching frequency of the frequency-controlled time constant. In this manner, the filter characteristic, or frequency response of the circuit can be changed as desired.

In the circuit of Fig. 1, the circuit is designed to sense QRS waves from a patient, and to insure that there are no artifacts due to stimulus pulses after the refractory period. By using a "restore" switch, the active filter bandpass characteristic is shifted to a higher frequency, and lower gain, during a predetermined time following the stimulus, e.g. 60 msec. The resulting effect of this switching is a fast recovery of the amplifier-filter combination, because of the shorter time constant during the recovery period. Depending upon the energy of the stimulus pulse delivered, an improvement of up to a factor of 2 can be achieved in the response time, which provides a substantial improvement in avoiding detection of artifacts due to the stimulus pulses.

Referring specifically to the circuitry, the input is provided at the terminal connected to resistor 51. The other side of 51 is coupled through capacitor 52 to the $V_{FF}$ line, and through capacitor 53 to point P; which is the input to an input amplifier comprised of FET transistors 55A, 55B, 55C and 55D. Transistors 55B and 55D provide a virtual 1 megohm resistance between node 0 and the $V_{FF}$ line. The output from the input amplifier is taken from transistor 55C and inputted to the common gates of transistors 58A, 58B, which together provide an inverting amplifier. The drain of transistor 58A is connected to current source 59, which provides 100 nA; the drain of 58B is connected to the output of current source 60, which provides 200 nA.

The output of the inverting amplifier, at node L, is connected in two paths. A feedback path goes through the circuit made up of 4 FETs 64A—D, to provide a feedback signal at node O, which is connected through resistor 57 to node P at the input to the input amplifier. The output circuit connected to node L comprises capacitor 65 in series with resistor 66, which in turn is connected through FET 70, the source of which is connected as the output terminal. The gate is connected to receive a "recover" signal, which gate switches 70 in or out of the circuit. When FET 70 is controlled to conduct, RC combination 65, 66 is in the circuit; when the switch is opened by the recover signal, there is no signal at the output and the circuit recovers quickly from any transients introduced by the stimulus signal.

For the circuit of Fig. 1, the following typical values are used for the resistors and capacitors:
Resistor 51—100 K ohm
 Capacitor 52—10 nano farads
Resistor 57—15 M ohm
 Capacitor 53—2.7 nano farads
Resistor 66—270 K ohm
 Capacitor 65—22 nano farads
The transconductance from node P to node L is about 15 nA/mV; from node L to node O about 1.0 nA/mV.

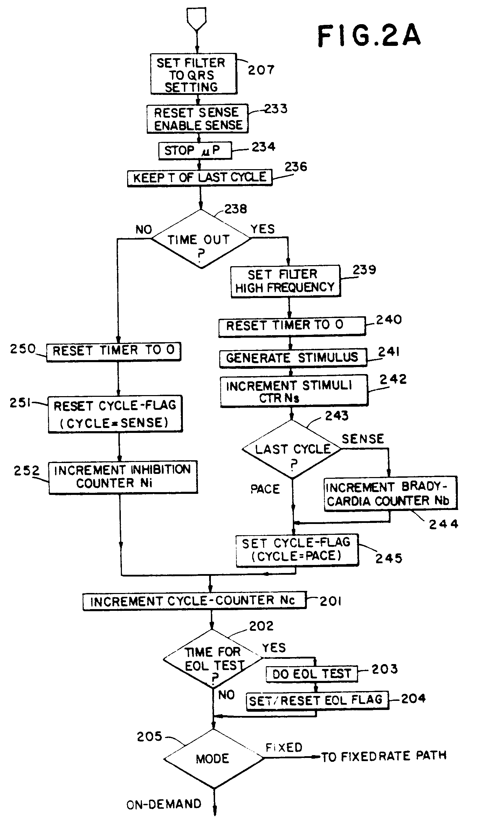

Referring to Fig. 2A, there is illustrated an example of a microprocessor controlled cardiac pacemaker which changes filter characteristics and/or sensitivity within the pacer cycle. The program, as illustrated, starts at a time just after the time out of the pacer refractory period. At block 207, the filter of the input amplifier, connected to receive a patient cardiac signal, is set to an appropriate QRS setting, i.e., set to an appropriate bandpass for detecting a patient QRS signal. At block 233, the microprocessor enables sensing through the input amplifier, and, for example, sets the sensitivity at 2 mV. At block 234, the microprocessor is stopped to await either timeout or a sensed QRS. When either of these events occurs, the microprocessor picks up at block 236 and records the time T of the last cycle, and then determines whether a timeout has occurred, at block 238.

If a timeout has occurred, meaning there has been no natural patient beat, the program block branches to the right. In preparation for generating the stimulus, the filter of the input amplifier is first set to a high frequency characteristic, at block 239, in order to quickly damp out any artifacts produced by the generated stimulus. Thereafter the pacer timer is set to zero at block 240, and the stimulus is generated at block 241. The pacer cycles through the remainder of the program, and the filter is reset to the normal QRS setting at block 207 during the next cycle.

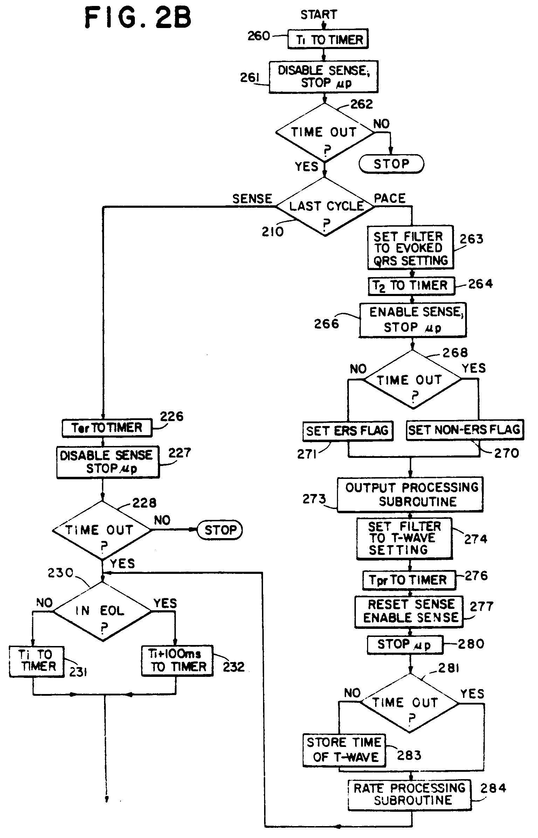

Referring to Fig. 2B, there is shown a block diagram of a further portion of a pacer control program providing for controlling the rate of delivered stimulus pulses as a function of sensed stimulus-T wave time intervals. Reference is made to U.S. Patents Nos. 4,228,803 and 4,305,396. At the top of Fig. 2B, the routine starts after stimulus delivery provided in Fig. 2A. A time delay is introduced at block 260, corresponding to the delay between the stimulus and the start of evoked response sensing. The sense amplifier is disabled at block 261, and the microprocessor is stopped to wait for the evoked response. At block 262, the microprocessor determines whether the timer has timed out. If yes, and the last cycle was a pace cycle, as determined at 210, the pacer control branches to the right. At block 263, a switchable filter such as that of Fig. 1 with a multiple of parallel output circuits is set to the evoked QRS setting, i.e., to a filter characteristic optimally designed to detect an evoked QRS. A period $T_2$ is put into the timer at 264, during which the pacer looks for the evoked response. The sense amplifier is enabled at block 266, for example with a sensitivity of 8 mV. At block 268, it is determined whether the timer has timed out. If no, meaning that an evoked response was detected, the ERS flag is set at block 271. If yes, meaning that there was no evoked response, the non-ERS flag is set at 270. Following this, at block 273, the microprocessor goes through the output processing subroutine, to change the stimulus magnitude if required to achieve heart capture. At block 274, the filter characteristic of the input amplifier is modified to a characteristic adapted for detecting the T wave portion of the heart signal. Following this, at block 276, a time interval $T_{PT}$ corresponding to the T wave time is set into the timer, and at block 277 the sense amplifier is enabled at a sensitivity of, for example, 1 mV. The microprocessor is stopped at 280, and is started again at 281 either by a sensed T wave or by timing out. If it is not timed out, meaning that a T wave was sensed, the time of this T wave in relation to the delivered pulse stimulus is stored at 283. At block 284, the microprocessor goes through a rate subprocessing routine to change the pacer rate, as set forth in U.S. Patent 4,228,803.

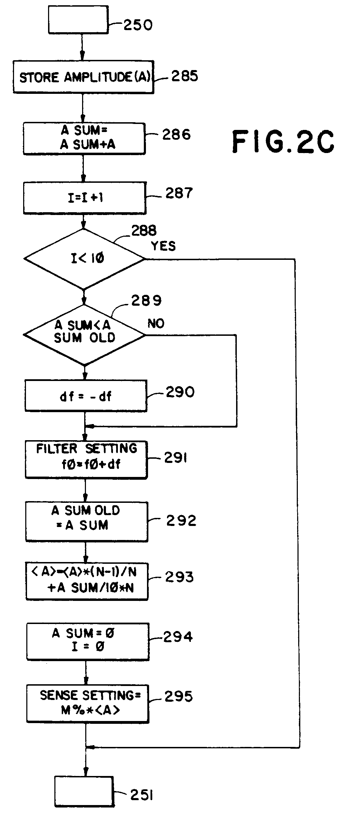

Referring to Fig. 2C, there is illustrated a routine for shifting the filter characteristic of a pacer input filter, as a function of monitored operation of the filter over a plurality of pacer cycles. This routine may be positioned between blocks 250 and 251 of Fig. 2A. While, in this example, the center frequency only of the filter is shifted, it is to be understood that a more complicated circuit modification can be utilized for shifting the lower and higher cut off points independently. The example of Fig. 2C is by way of illustration only, for showing switchable control of a circuit characteristic as a function of monitored circuit performance, i.e., a measured value of circuit operation, over a plurality of cycles.

Referring specifically to Fig. 2C, following block 250 the amplitude of the received input wave, QRS, P, etc., is stored at 285. At block 286, a new sum designated A SUM is accumulated by adding the just received amplitude A to the prior sum. At block 287, the number of iterations I is incremented by 1. In the illustration, the routine iterates 10 times in order to accumulate a sum, but it is to be understood that the number of iterations is a matter of choice. At block 288, it is determined whether I is less than 10. If yes, more cycles are to be measured, and the routine branches directly to block 251. If no, meaning that 10 measurements have now been accumulated, the program proceeds to block 289, where it is determined whether A SUM is less than the prior, or old A SUM. If no, meaning that A SUM has increased, the routine branches to block 291. If yes, the routine first performs the operation at block 290 of changing the incremental frequency deviation from +df to -df. Note that at block 289 the program is checking to see whether the new cumulative amplitude has improved or not relative to the previous cumulative amplitude. If yes, then at block 291 the filter setting is changed in the same direction by the small step df, by setting the new center frequency fO to fO+df. If the cumulative amplitude is less, then it is presumed that the center frequency should be shifted in the other direction, which is accomplished by making df= -df. Then, at block 292, the microprocessor stores the cumulated A SUM as A SUM OLD. At block 293, the mean amplitude ⟨A⟩ is updated by a predetermined algorithm which is a matter of choice. Following this, at block 294, the present values of A SUM and I are set to 0. At block 295, the sensitivity setting is automatically re-programmed to a fixed safe percentage M of the measured mean amplitude. Following this, the routine proceeds to block 251, and continues.

There have thus been set forth examples of a preferred embodiments of the invention, for microprocessor control of switchable characteristics of 1 or more circuits in a device such as a cardiac pacemaker. Reference is made to U.S. Patent No. 4,305,396 for examples of other signal characteristics which may be measured by a cardiac pacemaker, and for which specific circuit characteristics are switched into and out of operation. Although a microprocessor embodiment has been shown by way of illustration, it is understood that other equivalent digital or analog means may be incorporated into a device in accordance with this invention, providing switchable circuit characteristics and control therefor. As used in the claims, the term "circuit characteristic" means frequency response, sensitivity, gain, etc., and it does not include an operating parameter such as time set in a timing circuit.

## Claims

1. A cardiac pacemaker having

   - an input amplifier (Fig. 1) for amplifying sensed cardiac signals,
   - said input amplifier having multiple parallel output circuits which each comprises a switch (70) and a filter (65, 66) having a particular frequency response characteristic enabling a particular frequency characteristic of said input amplifier and thus a particular signal response at the corresponding output while the corresponding switch is activated,
   - microprocessor control means (Fig. 2B) for controlling the frequency characteristic of said input amplifier on a cyclical basis to have respective different frequency characteristics during sensing of evoked QRS complex (263) and T wave (274) portions of each sensed cardiac signal, and
   - means (Fig. 2C) for monitoring a particular signal response of said input amplifier over a predetermined number of unevoked cycles and determining a measure of the amplitudes of said response over said cycles (285, 286, 287),
   - said microprocessor control means comprising means (Fig. 2C) for shifting the corresponding particular frequency characteristic of the input amplifier as function of said measure (289, 290, 291).

2. The cardiac pacemaker as described in claim 1, having means for measuring stimulus-T wave time during each pacing cycle (283), and means for controlling pacing stimulus rate as a function of said measured time (284).

## Patentansprüche

1. Herzschrittmacher mit

   - einem Eingangsverstärker (Fig. 1) zum Verstärken erfaßter Herzsignale,
   - wobei der Eingangsverstärker mehrere parallele Ausgangsschaltungen aufweist, die jeweils einen Schalter (70) und ein Filter (65,66) mit einer bestimmten Frequenzantwortcharakteristik aufweisen, wodurch eine bestimmte Frequenzcharakteristik des Eingangsverstärkers und so eine bestimmte Signalantwort am entsprechenden Ausgang ermöglicht wird, wenn der entsprechende Schalter aktiviert ist,

- einer Mikroprozessor-Steuerungseinrichtung (Fig. 2B) zum Steuern der Frequenzcharakteristik des Eingangsverstärkers auf zyklischer Basis, wodurch entsprechende verschiedene Frequenzcharakteristika während des Erfassens evozierter QRS-Komplex-(263) und T-Zacken-Teile (274) eines jeden Herzsignals ermöglicht werden, und

- einer Einrichtung (Fig. 2C) zum Überwachen einer bestimmten Signalantwort des Eingangsverstärkers über eine vorbestimmte Anzahl unevozierter Zyklen und zum Bestimmen einer Größe der Amplituden der Antworten während der Zyklen (285, 286, 287),

- wobei die Mikroprozessor-Steuerungseinrichtung eine Einrichtung (Fig. 2C) zum Verschieben der entsprechenden bestimmten Frequenzcharakteristik des Eingangsverstärkers in Abhängigkeit von der Größe (289, 290, 291) aufweist.

2. Herzschrittmacher nach Anspruch 1, mit einer Einrichtung zum Messen der Stiumulus-T-Zacken-Zeit während eines jeden Schrittmacherzyklus (283) und einer Einrichtung zum Steuern der Schrittmacher-Stimulierfrequenz in Abhängigkeit von der gemessenen Zeit (284).

**Revendications**

1. Stimulateur cardiaque comprenant :

- un amplificateur d'entrée (Fig. 1) pour amplifier les signaux cardiaques décelés

- ledit amplificateur d'entrée comprenant de multiples circuits parallèles de sortie ayant chacun un commutateur (70) et un filtre (65, 66) possédant une caractéristique particulière de réponse de fréquence permettant une caractéristique particulière de fréquence dudit amplificateur d'entrée et donc une réponse, formée d'un signal particulier, à la sortie correspondante pendant que le commutateur respectif est activé,

- des moyens de commande à microprocesseur (Fig. 2B) pour commander la caractéristique de fréquence dudit amplificateur d'entrée, sur une base cyclique, en vue d'obtenir des caractéristiques différentes respectives de fréquence pendant la détection du complexe QRS évoqué (263) et les parties d'onde T (274) de chaque signal cardiaque décelé et

- des moyens (Fig. 2C) pour surveiller une réponse, formée d'un signal particuliers dudit amplificateur d'entrée sur un nombre fixé à l'avance de cycles non-évoqués et pour déterminer une mesure des amplitudes de ladite réponse sur lesdits cycles (285, 286, 287),

- lesdits moyens de commande à microprocesseur comprenant des moyens (Fig. 2C) pour déplacer la caractéristique de fréquence particulière correspondante de l'amplificateur d'entrée en fonction de ladite mesure (289, 290, 291).

2. Stimulateur cardiaque tel que décrit à la revendication 1, comprenant des moyens pour mesurer la durée d'onde du stimulus T pendant chaque cycle de stimulation (283) et des moyens pour commander le rythme du stimulus de stimulation en fonction de ladite durée mesurée (284).

FIG.1

# FIG.2A

SET FILTER TO QRS SETTING — 207

RESET SENSE ENABLE SENSE — 233

STOP μP — 234

KEEP T OF LAST CYCLE — 236

238 — TIME OUT ?
NO / YES

SET FILTER HIGH FREQUENCY — 239

RESET TIMER TO O — 240

250 — RESET TIMER TO O

GENERATE STIMULUS — 241

INCREMENT STIMULI CTR Ns — 242

251 — RESET CYCLE-FLAG (CYCLE = SENSE)

243 — LAST CYCLE ?
PACE / SENSE

252 — INCREMENT INHIBITION COUNTER Ni

INCREMENT BRADY-CARDIA COUNTER Nb — 244

SET CYCLE-FLAG (CYCLE = PACE) — 245

INCREMENT CYCLE-COUNTER Nc — 201

202 — TIME FOR EOL TEST ?
YES / NO

DO EOL TEST — 203

SET/RESET EOL FLAG — 204

205 — MODE
FIXED — TO FIXED RATE PATH
ON-DEMAND

# FIG. 2B

FIG.2C